# EUROPÄISCHE PATENTANMELDUNG

(11) **EP 1 327 463 A1**
(43) Veröffentlichungstag der Anmeldung: **16.07.2003**
(21) Anmeldenummer: 02029100.1
(22) Anmeldetag: 30.12.2002
(51) Int. Cl.: A61P 11/06, A61K 9/06, A61K 31/495, A61K 31/4545

(54) **Gel enthaltend Cetirizin und Loratadin zur topischen Anwendung**

(30) Priorität: 04.01.2002 EP 02000131
(71) Anmelder: Oramon Arzneimittel GmbH, 88471 Laupheim (DE)
(72) Erfinder: Walch, Hatto, Dr., 76534 Baden Baden (DE)
(74) Vertreter: Schwabe - Sandmair - Marx

(57) **Zusammenfassung**

Die vorliegende Erfindung betrifft eine Arzneimittelzubereitung, enthaltend die nicht sedierenden H₁-Antihistaminika Cetirizin, Loratadin, Mischungen oder pharmazeutisch annehmbare Salze derselben, für die topische Anwendung in Form eines Gels.

## Beschreibung

Die vorliegende Erfindung betrifft eine Arzneimittelzubereitung, enthaltend die nicht sedierenden H₁-Antihistaminika Cetirizin, Loratadin, Mischungen oder pharmazeutisch annehmbare Salze derselben, für die topische Anwendung in Form eines Gels.

### STAND DER TECHNIK

Histamin ist ein biogenes Amin, das durch die Decarboxylierung der Aminosäure Histidin entsteht und als Gewebshormon im menschlichen und tierischen Organismus, insbesondere in der Haut und Lunge, vorkommt. Es ist aber beispielsweise auch im Bienengift, im Speicheldrüsensekret stechender Insekten, in Brennnesseln und anderem enthalten. Im menschlichen Körper wird Histamin in den basophilen Granulozyten und den Mastzellen gespeichert. Bei allergischen Hautreaktionen wird überschießend Histamin ausgeschüttet und ist dann mit verantwortlich für das Jucken und die Quaddelbildung bzw. Rötungen der Haut. Diese Ausschüttung erfolgt bei jeder Schädigung menschlicher und tierischer Zellen und verursacht Reaktionen des umgebenden Gewebes. Obwohl Histamin im menschlichen Körper sehr schnell metabolisiert wird, können die durch die Ausschüttung hervorgerufenen Wirkungen über einen längeren Zeitraum anhalten, was zu einer erheblichen Beeinträchtigung des Wohlbefindens der betroffenen Person führt. Derzeit werden zur Behandlung dieser Wirkungen der Histaminausschüttungen Antihistaminika vor allem auf oralem Wege verabreicht.

Unter den Antihistaminika versteht man Pharmaka, die Histamin kompetitiv von dessen Rezeptoren verdrängen und dadurch die Histaminwirkungen aufheben können. Ihrem Angriff an den verschiedenen Histaminrezeptoren entsprechend, unterscheidet man hierbei H₁-Antihistaminika und H₂-Antihistaminika. Neben ihren antihistaminischen Eigenschaften besitzen fast alle H₁-Antihistaminika zusätzlich eine spasmolytische und lokalanästhetische Wirkung. Am zentralen Nervensystem zeigen die meisten Präparate weiterhin einen sedierenden Effekt.

Die H₁-Antihistaminika sind üblicherweise indiziert bei allen Erkrankungen, die auf einer Freisetzung von Histamin beruhen, wobei Substanzen mit stärker sedierender Wirkung auch als Antiemetica oder Schlafmittel verwendet werden. In jedem Fall ist die bedeutsamste Nebenwirkung die Beeinflussung des zentralen Nervensystems, das heißt der sedierende Effekt. Weil dieser sedierende Effekt einer allgemeinen Anwendung, beispielsweise bei Erkältungen oder Heuschnupfen, entgegenstand, wurden in jüngerer Zeit H₁-Antihistaminika der zweiten Generation mit geringer sedierenden Nebenwirkungen entwickelt. Diese werden üblicherweise und im Rahmen der vorliegenden Anmeldung als "nicht sedierende H₁-Antihistaminika" bezeichnet. Typische Beispiele für die nicht sedierenden H₁-Antihistaminika sind Terfenadin, Astemizole, Antazolin, Cetirizin, Loratadin, Ketotifen, Acrivastin, Ebastin, Efletirizin, Epinastin, Fexofenadin, Levocabastin, Mizolastin, Oxatomide, Sebastin, Temelastin und Azelastin.

Typischerweise werden H₁-Antihistaminika bspw. zur Behandlung des Heuschnupfens oral und gelegentlich nasal, in Form von Sprays, verabreicht. Bevorzugt verwendete Wirkstoffe sind Cetirizin oder Loratadin.

Gemäß der amerikanischen Drugdex Drug Evaluations ist Cetirizin, das ein Metabolit des Hydroxyzins ist, ein H₁-Antagonist mit nicht sedierenden Eigenschaften. Die empfohlene orale Dosis für Cetirizin beträgt 10 mg einmal täglich für Erwachsene oder 2,5 bis 5 mg einmal täglich für Kinder. Cetirizin wird üblicherweise zur Behandlung des Heuschnupfens, der allergischen Rhinitis, der chronischen Urtikaria und von Asthma eingesetzt. Die Verabreichung erfolgt meist oral. Ebenfalls bekannt ist die intranasale Anwendung eines Cetirizin-Sprays, obwohl entsprechende Formulierungen kommerziell nicht erhältlich sind.

US-A-4,525,358 beschreibt die Herstellung von Cetirizin und anderer Hydroxizin-Derivate wie auch die Verwendung als antiallerges, antihistamines, bronchiodilatorisches und antispasmodisches Mittel. In allgemeiner Form beschreibt das Dokument weiterhin eine mögliche orale, parenterale oder topische Verwendung der offenbarten Hydroxyzin-Derivate. Beispiele für eine topische Formulierung werden jedoch nicht gegeben. Insgesamt ist Cetirizin bis zur Gegenwart ausschließlich oral oder versuchsweise intranasal verabreicht worden, und zwar für die oben genannten Indikationen.

Ähnlich werden auch andere H₁-Antihistaminika der zweiten Generation, wie beispielsweise das Loratadin, in der Praxis lediglich oral formuliert und verabreicht. Bei Loratadin handelt es sich gemäß dem amerikanischen Drugdex Drug Evaluations ebenfalls um ein nicht sedierendes Antihistaminikum. Der Wirkstoff wird für Erwachsene in einer oralen Dosis von 10 mg einmal täglich und für Kinder in einer Dosis von 5 mg einmal täglich verabreicht. Außerdem bekannt sind orale Kombinationsprodukte, beispielsweise mit Pseudoephedrin. Indikationsgebiete für die Verwendung des Loratadins sind, wie für das Cetirizin, die allergische Rhinitis, das Asthma sowie die chronische idiopathische Urtikaria.

Die Herstellung der Substanz ist im US-Patent 4,282,233 beschrieben, die auch pharmazeutische Zubereitungen zur enteralen oder parenteralen Verabreichung beschreibt, nicht jedoch die topische Verabreichung des Wirkstoffes.

Die Europäische Patentanmeldung 0 903 151 beschreibt die Verwendung von Kombinationen aus nicht sedierenden Antihistaminika und α-adrenergen Wirkstoffen für die topische Behandlung der Rhinitis bzw. Conjunctivitis und von Erkältungssymptomen. Die beschriebenen topischen Zubereitungen enthalten das Antihistaminikum in einer Menge von 0,001 bis 0,5 %, vorzugsweise 0,05 % bis 0,1 %. Darüber hinaus beschreibt die Anmeldung ausschließlich topische Formulierungen zur Verabreichung über die Schleimhäute, nämlich Nasensprays, Nasentropfen oder Augentropfen. Eine topische Anwendung auf der Haut ist nicht offenbart oder intendiert.

Die WO 01/87 236 offenbart ein Spray, enthaltend eine pharmakologisch aktive Verbindung wie ein Antihistaminikum, zur Behandlung von Insektenbissen, Insektenstichen, Nesselsucht, der atopischen Dermatitis und der Kontaktdermatitis, wie auch Sonnenbrand, Neurodermatitis, Urtikaria und Ekzem. Gleichzeitig offenbart das Dokument Hydroxizin enthaltende Cremes, Gele, Schäume, Salben oder Lotionen. Details dieser Zubereitungsformen sind nicht angegeben.

Die US-A-5,993,833 offenbart topische Zubereitungen, die ein Antihistaminikum, einen Interleukin-1-Antagonisten und/oder einen TNFα-Antagonisten enthalten. Diese Zubereitungen können in allen möglichen topischen Formen unter anderem wasserfreien oder lipophilen Gelen, Cremes, Emulsionen, Schäumen oder ähnlichem vorliegen. Das Dokument lehrt jedoch eher den Zusatz eines Antihistaminikums zu einer kosmetischen, dermatologischen oder pharmazeutischen Zubereitung als eine solche Zubereitung selbst, wobei nicht das Antihistaminikum der eigentliche Wirkstoff ist, sondern letzteren nur verträglicher machen soll.

Als Bestandteile der Zubereitungen sind alle üblichen Bestandteile wie Öle, Emulgatoren, Lösungsmittel (niedere Alkohole und Propylenglykol), hydrophile Geliermittel (Carbomer, Acrylcopolymere, Polysacharide wie Hydroxypropylcellulose, wie auch lipophile Geliermittel wie Ethylcellulose und Polyethylen) genannt. Die Beispiele veranschaulichen die Verwendung eines Antihistaminikums Cetirizin, Loratadin als Creme, Gel oder Lotion.

Die WO 01/39 774 offenbart eine Ketotifen enthaltende Zubereitung, die zur Behandlung von allergischen Zuständen, eingeschlossen Insektenbisse und -stiche, Nesselfieber, der atopischen und Kontaktdermatitis sowie Ekzem, Urtikaria, Neurodermitis, trockener Haut und Sonnenbrand, verwendet werden kann und in Form eines Gels, einer Lotion, eines Sprays, eines Schaums, einer Creme, einer Salbe und verschiedener Flüssigkeiten vorliegen kann. Obwohl die Zubereitungen verschiedene übliche Bestandteile enthalten können, sind Gele nicht besonders bevorzugt. Bevorzugt ist stattdessen eine Cremezubereitung Die EP 0 484 529 offenbart eine W/O-Hautcremezubereitung, umfassend einen aktiven Bestandteil wie unter anderem Ketotifen, und eine Cremebasis, welche einen Diglycerolfettsäureester und/oder Sorbitanfettsäureester mit einem HLB von 3 bis 7, ein mehrwertiges Metallsalz einer Fettsäure mit 10 bis 22 Kohlenstoffatomen, ein Salz einer anorganischen oder organischen Säure, eine Ölphasenkomponente und Wasser enthält.

Die oben beschriebene orale Verabreichung hat den Nachteil des langsamen Wirkungseintritts. Weiterhin sind mit der oralen Verabreichung die bekannten, sedierenden Nebenwirkungen verbunden, da der Wirkstoff gleichmäßig über den gesamten Organismus verteilt wird. Diese sind auch bei den H₁-Antihistaminika der zweiten Generation, wenn auch in abgeschwächter Form, vorhanden. Insbesondere in Situationen, in denen die volle Aufmerksamkeit des Betroffenen gefordert ist, wie beim Fahren eines Kfz, der Bedienung komplizierter Maschinen usw., stellt die Sedierung ein gewisses Gefährdungspotenzial dar.

Topisch werden die Wirkungen des Histamins auf der Haut gegenwärtig beispielsweise durch Auftrag von Isoprenalinsulfat, Benzocain, Diphenhydramin oder Benzalkoniumchlorid erreicht. Von diesen stellt das Diphenhydramin zwar ein Antihistaminikum dar, jedoch mit stark sedierender Wirkung. Gerade auf Grund dieser stark sedierenden Wirkung wird Diphenhydramin auch als Hypnotikum oder Sedativum verwendet.

Die topische Verabreichung der o. g. Substanzen hat entweder den Nachteil, dass sie nicht ausreichend spezifisch auf die Ursache der Erkrankung bzw. Störung, nämlich die Histaminausschüttung, wirkt und lediglich die Symptome beispielsweise durch Betäubung des Juckreizes, bekämpft werden. Umgekehrt kann die Anwendung von Diphenhydramin zusätzlich mit sedierenden Nebenwirkungen behaftet sein, wird es doch eher als Lokalanästhetikum verwendet. Darüber hinaus ist der Wirkungseintritt relativ langsam.

Ebenfalls bekannt sind die oben beschriebenen topischen Formulierungen von Ketotifen, Cetirizin und Loratadin. Diese weisen jedoch den Nachteil auf, dass der Wirkstoff schlecht durch die Haut penetriert und damit der Wirkungseintritt verzögert ist.

Es ist daher Aufgabe der Erfindung, eine schnell und lokal wirkende Arzneimittelform der bevorzugten Antihistaminika Cetirizin und Loratadin zur Therapie von Störungen oder Erkrankungen der Haut bereitzustellen, die auf einer übermäßigen Histaminausschüttung beruhen, welche einen schnellen Wirkungseintritt gestattet.

### ZUSAMMENFASSUNG DER ERFINDUNG

Die vorliegende Erfindung betrifft eine Arzneimittelzubereitung zur topischen Anwendung in Form eines Gels, enthaltend 0,05 bis 0,5 Gew.%, vorzugsweise 0,5 bis 5,0 Gew.-% eines nicht sedierenden H₁-Antihistaminikums, ausgewählt aus der Gruppe, bestehend aus Cetirizin, Loratadin und Mischungen und pharmazeutisch annehmbaren Salzen derselben, und mindestens einen Arzneimittelträger oder -hilfsstoff, der ein Lösungsmittel für das Antihistaminikum darstellt.

Weitere vorteilhafte Ausgestaltungen der Erfindung sind in den Ansprüchen angegeben.

### GENAUE BESCHREIBUNG DER ERFINDUNG

Es wurde nun überraschend gefunden, dass die obigen Aufgaben gelöst und die Nachteile des Standes der Technik überwunden werden können durch eine Arzneimittelzubereitung zur topischen Anwendung in Form eines Gels, enthaltend 0,05 bis 5,0 Gew. %, vorzugsweise 0,5 bis 5,0 Gew.-% eines nicht sedierenden H₁-Antihistaminikums, ausgewählt aus der Gruppe, bestehend aus Cetirizin, Loratadin und Mischungen und pharmazeutisch annehmbaren Salzen derselben, und mindestens einen Arzneimittelträger oder -hilfsstoff, der ein Lösungsmittel für das Antihistaminikum darstellt.

Die topische Anwendung der nicht sedierenden H₁-Antihistaminika Cetirizin und/oder Loratadin in Form eines erfindungsgemäßen Gels hat zum einen den Vorteil, dass der Wirkstoff dem lokal ausgeschütteten Histamin selbst durch kompetitive Bindung an den entsprechenden Rezeptor entgegenwirkt, die Symptome also weitgehend vermieden bzw. verringert werden können. Weiterhin zeigte sich bei den erfindungsgemäßen Formulierungen überraschend ein unerwartet schnellerer Wirkungseintritt gegenüber bekannten topischen Formulierungen.

Ohne hierdurch gebunden sein zu wollen, könnte dies auf der Tatsache beruhen, dass der Wirkstoff in den Formulierungen der Erfindung eben nicht nur suspendiert ist, sondern in Form einer echten Lösung vorliegt. Die sich dadurch ergebende molekulare Verteilung könnte wiederum die Resorptionsgeschwindigkeit erhöhen.

Die erfindungsgemäßen Gele sind vorzugsweise bestimmt zur Therapie einer Erkrankung oder Störung, ausgewählt aus der Gruppe, bestehend aus der Urtikaria, allergisch bedingten Dermatosen (allergischen Hautreaktionen), dem atopischen Ekzem, Juckreiz, Rötungen, Sonnenbrand und Insektenstichen.

Die erfindungsgemäßen Gele gestatten bei diesen meist großflächig auftretenden Störungen ein leichtes Spreiten der Formulierung auf der Haut und haben ggf. einen vom Patienten als angenehm empfundenen kühlenden Effekt. Die Resorption kann erfindungsgemäß auch noch dadurch beschleunigt werden, dass der Wirkstoff in einem leicht brechenden Gel formuliert wird, das diesen auf der Haut schneller freisetzt.

Die erfindungsgemäßen Arzneimittelzubereitungen enthalten, wie oben beschrieben, ein nicht sedierendes H₁-Antihistaminikum, ausgewählt aus der Gruppe, bestehend aus Cetirizin, Loratadin, Mischungen und pharmazeutisch annehmbaren Salzen derselben. Bei den pharmazeutisch annehmbaren Salzen kann es sich beispielsweise um das Hydrochlorid, Hydrobromid, Hydrojodid, Citrat, Carbonat, Malat, Tartrat usw. handeln. Ebenso können erfindungsgemäß Mischungen von Cetirizin und Loratadin und/oder ihrer Salze eingesetzt werden.

Die Gele enthalten das nicht sedierende H₁-Antihistaminikum und/oder ein pharmazeutisch annehmbares Salz desselben, in einer Menge von 0,05 bis 5,0 Gew. %, vorzugsweise 0,5 bis 5,0 Gew.-%. Stärker bevorzugt sind in der Formulierung 0,8 bis 1,5 Gew.-% an Wirkstoff enthalten.

Die erfindungsgemäßen Gele enthalten zusätzlich einen oder mehrere Arzneimittelhilfsund Trägerstoffe. Diese Arzneimittelhilfs- und Trägerstoffe sind vorzugsweise ausgewählt aus für die Formulierung der oben genannten Zubereitungsform eines Gels bekannten und geeigneten Arzneimittelhilfs- und Trägerstoffen. Mindestens einer der Hilfs- und Trägerstoffe stellt ein Lösungsmittel für den verwendeten Wirkstoff dar. Als mögliche Arzneimittelhilfs- und Trägerstoffe sind zu nennen Lösungsmittel wie Wasser, Alkohole, insbesondere C₁-C₅-Alkohole wie Ethanol, n-Propanol, 2-Propanol, Isopropanol, t-Butylalkohol, Ether wie MTBE, Ketone wie Aceton, Methylethylketon; Feuchthaltemittel wie Glycerol, Glykole wie Ethylenglykol, Propylenglykol; Emulgatoren wie partiell mit langkettigen (C₁₂-C₂₄-) Fettsäuren veresterte ggf. mehrwertige niedere C₁₋₅-Alkohole wie z.B. Glycerolmonostearat, Isopropylmyristat, Fettsäureester von Zuckeralkoholen wie Sorbitanmonofettsäureester, polyethoxylierte Derivate dieser Verbindungen, Polyethoxyethylenfettsäureester und -fettalkoholether, Cholesterin, Cetylstearylalkohol, Wollwachsalkohole sowie synthetische Tenside mit niedrigem HLB-Wert; Lösungsvermittler wie Carbopol; dünnflüssige Paraffine, Triglyceride; lipophile Substanzen wie Isopropylmyristat; pH-Regulatoren wie TEA, Carbonate und Phosphate; Chelatbildner wie EDTA und dessen Salze; und Konservierungsmittel.

Neben diesen Arzneimittelhilfs- und Trägerstoffen können die erfindungsgemäßen Arzneimittelzubereitungen zusätzlich einen oder mehrere kosmetische Hilfsstoffe enthalten. Derartige kosmetische Hilfsstoffe sind dem Pharmazeuten bekannt. Sie können insbesondere aus der Gruppe ausgewählt werden, bestehend aus Feuchthaltemitteln, Glättungsmitteln, UV-Filtern, Pigmenten, Farbstoffen, Parfümen, Vitaminen und Bleichmitteln. Besonders bevorzugt sind Feuchthaltemittel, Glättungsmittel und Parfüme.

Bezüglich der erfindungsgemäß zu verwendenden Gele sind hydrophile Gele und hydrophobe Gele zu unterscheiden. Beide können erfindungsgemäß verwendet werden, gegebenenfalls in Abhängigkeit von der zu therapierenden Indikation. Bei den hydrophilen Gelen handelt es sich bekanntermaßen um Gele mit einem hohen Wassergehalt (80-90 %).

Bei den hydrophoben Gelen oder so genannten Oleogelen handelt es sich um fettende Gele, die mit flüssigen Paraffinen, Polyethylenglykolen, Triglyceriden usw. als Basis in Verbindung mit Gelbildnern wie hochdispersem Siliziumdioxid oder Aluminium- oder Zinkseifen hergestellt werden. Hydrophile Gele sind erfindungsgemäß insbesondere für die Indikationen Insektenstiche und Sonnenbrand geeignet, wogegen Oleogele insbesondere beim atopischen Ekzem zum Einsatz kommen können.

Erfindungsgemäß ganz besonders bevorzugt ist nach einer ersten Ausführungsform eine Gelzubereitung, enthaltend
0,05 - 5 Gew.%, bevorzugt 0,5-5 Gew.-%, noch stärker bevorzugt 0,8-1,5 Gew.-% Antihistaminikum (vorzugsweise Cetirizinhydrochlorid, Cetirizin, Loratadinhydrochlorid, Loratadin),
1-2 Gew.-% Lösungsvermittler, insbesondere Carbopol,
0-1 Gew.-%, vorzugsweise 0-0,4 Gew.-% Chelatbildner, insbesondere EDTA,
20-40 Gew.-%, vorzugsweise 30-40 Gew.-% Alkylenglykol, insbesondere Propylenglykol,
5-20 Gew.-%, vorzugsweise 10-20 Gew.-% Alkohol, insbesondere n-Propanol, Isopropanol, 0-4 Gew.-%, vorzugsweise 1-4 Gew.-% pH-Regulator, insbesondere Triethanolamin, und
eine Restmenge ad 100 %, d.h. 20-70 Gew.-%, vorzugsweise 30-60 Gew.-%, am meisten bevorzugt 35-50 Gew.-% gereinigtes Wasser, wobei sich die Gewichtsanteile der Bestandteile auf 100 Gew.-% addieren und auf das Gesamtgewicht der Zubereitung bezogen sind.

Gemäß einer zweiten bevorzugten Ausführungsform wird erfindungsgemäß ein Gel bereit gestellt, enthaltend
0,05 - 5 Gew.-%, vorzugsweise 0,5-5 Gew.-%, noch stärker bevorzugt 0,8-1,5 Gew.-% Wirkstoff (vorzugsweise Cetirizinhydrochlorid, Cetirizin, Loratadinhydrochlorid, Loratadin),
5-20, vorzugsweise 10 bis 15 Gew.-% Alkylenglykol, insbesondere Propylenglykol,
2-10 Gew.-% vorzugsweise 5-10 Gew.-% ethoxylierte Partialglyceride von Fettsäuren, insbesondere mittelkettigen Fettsäuren, wie das unter der Marke Softigen® 767 vertriebene Produkt,
3-10 Gew.-%, vorzugsweise 3-5 Gew.-% Cellulosederivate insbesondere Celluloseether wie Metholose und
eine Restmenge ad 100 %, d.h. 50-90 Gew.-%, vorzugsweise 55-90 Gew.-% und am meisten bevorzugt 80-90 Gew.-% gereinigtes Wasser, wobei sich die Gewichtsanteile der oben genannten Substanzen auf 100 Gew.-% addieren und auf das Gesamtgewicht der Zubereitung bezogen sind.

Gemäß einer dritten bevorzugten Ausführungform wird ein Gel bereitgestellt, enthaltend:
0,05 - 5 Gew.-%, vorzugsweise 0,5-5 Gew.-%, noch stärker bevorzugt 0,8-1,5 Gew.-% Wirkstoff (vorzugsweise Cetirizinhydrochlorid, Cetirizin, Loratadinhydrochlorid, Loratadin),
0,1-2 Gew.-%, vorzugweise 0,6 - 1,0 Gew.-% Lösungsvermittler, insbesondere Carbopol,
0,2625 - 0,4375 Gew.-%, vorzugsweise 0,3 - 0,4 Gew.-% einer mindestens 25 %igen wässrigen Ammoniaklösung,
1,5 - 2,5 Gew.-%, vorzugsweise 1,8 - 2,2 Gew.-% eines Esters einer C₁₀₋₂₀-Fettsäure mit einem niederen C₂₋₅-Alkohol, insbesondere Isopropylmyristat,
4,5 - 7,5 Gew.-%, vorzugsweise 5,5 - 6,5 Gew.-% Cetiol V,
0,0375 - 0,0625 Gew.-%, vorzugsweise 0,045 - 0,055 Gew.-% Chelatbildner, insbesondere Na-EDTA,
2,25 - 3,75 Gew.-%, vorzugsweise 2,75 - 3, 25 Gew.-% Macrogol 400,
15,0 - 25,0 Gew.-%, vorzugsweise 17,0 - 22,0 Gew.-% eines niederen sekundären C₂₋₅-Alkohols, insbesondere 2-Propanol, und
eine Restmenge ad 100 %, d.h. 47,1625 - 83,9375 Gew.-%, vorzugsweise 55 - 65 Gew.-% gereinigtes Wasser, wobei sich die Gewichtsanteile der oben genannten Substanzen auf 100 Gew.-% addieren und auf das Gesamtgewicht der Zubereitung bezogen sind.

Bei letzterem Gel handelt es sich um eine besonders schnell auf der Haut brechende Formulierung, die daher eine sehr gute Penetration gestattet.

Die erfindungsgemäße Verabreichungsform eines Gels gestattet die Vermeidung des systemischen Umwegs und gestattet es außerdem, erheblich geringere Mengen an Wirkstoff als herkömmlich erforderlich und für die orale Therapie als notwendig beschrieben, zu verabreichen. Beispielsweise werden bei einem Auftrag von 200 mg (ca. 200 µl) Gel mit einer Gewichtskonzentration von 0,5 Gew.-% Wirkstoff, 1 mg Wirkstoff verabreicht.

Die Erfindung soll nun anhand der folgenden Beispiele weiter veranschaulicht werden. Diese dienen lediglich zur Veranschaulichung und sollen die Erfindung nicht beschränken.

### BEISPIELE

### Beispiel 1: Gelzubereitung

Aus den folgenden Bestandteilen wurde eine Gelzubereitung auf bekannte Weise hergestellt:

| | Gramm |
|---|---|
| Cetirizin-HCl | 1,00 |
| Carbopol (Aerylat) | 1,25 |
| EDTA | 0,10 |
| Propylenglykol | 34,00 |
| n-Propanol | 15,00 |
| Triethanolamin | 1,67 |
| gereinigtes Wasser | 45,98 |
| | 100,00 |

Die Gelzubereitung gemäß Beispiel 1 wurde an 20 freiwilligen Probanden, die an Juckreiz nach Insektenstichen bzw. Sonnenbrand litten, geprüft. Dabei zeigte sich, dass sowohl Sonnenbrand als auch Juckreiz und Rötungen rasch abklangen. Daneben war das Gel gut hautverträglich.

### Beispiel 2: Gelzubereitung

Aus den folgenden Bestandteilen wurde auf herkömmliche Weise eine Gelzubereitung hergestellt:

| | Gramm |
|---|---|
| Cetirizin-HCl | 1,00 |
| Propylenglykol | 10,00 |
| Softigen® 767 | 5,00 |
| Metolose | 3,00 |
| gereinigtes Wasser | 81,00 |
| | 100,00 |

### Beispiel 3: Gelzubereitung

Aus den folgenden Bestandteilen wurde auf herkömmliche Weise eine Gelzubereitung hergestellt:

| | Gramm |
|---|---|
| Loratadin-HCl | 1,00 |
| Carbopol | 0,800 |
| 25%ige Ammoniaklösung | 0,350 |
| Isopropylmyristat | 2,000 |
| Cetiol V | 6,000 |
| Paraffin fl. | 2,000 |
| Na-EDTA | 0,050 |
| Macrogol 400 | 3,000 |
| 2-Propanol | 20,000 |
| gereinigtes Wasser | 65,550 |
| | 100,00 |

## Patentansprüche

1. Arzneimittelzubereitung zur topischen Anwendung in Form eines Gels, enthaltend 0,05 - 5,0 Gew.-% eines H1 Antihistaminikums ausgewählt aus der Gruppe bestehend aus Cetirizin, Loratadin und Mischungen derselben, sowie mindestens einen Arzneimittelträger oder -hilfsstoff, der ein Lösungsmittel für das H1 Antihistaminikum darstellt.

2. Arzneimittelzubereitung nach Anspruch 1, enthaltend:
0,05 - 5,0 Gew.-% des H1 Antihistaminikums, ausgewählt aus der Gruppe bestehend aus Cetirizin, Loratadin und Mischungen derselben,
1,0 - 2,0 Gew.-% Carbopol,
0 - 0,4 Gew.-% EDTA,
20 - 40 Gew.-% n-Propanol,
10 - 20 Gew.-% TEA, und
Wasser ad 100 %.

3. Arzneimittelzubereitungnach Anspruch 1, enthaltend:
0,05 - 5,0 Gew.-% des H1 Antihistaminikums, ausgewählt aus der Gruppe bestehend aus Cetirizin, Loratadin und Mischungen derselben,
5 - 20 Gew.-% Propylenglykol,
2 - 10 Gew.-% ethoxyliertes partielles Glycerid mittelkettiger Fettsäuren,
3 - 10 Gew.-% Celluloseether, und
Wasser ad 100 %.

4. Arzneimittelzubereitungnach Anspruch 1, enthaltend:
0,05 - 5,0 Gew.-% des H1 Antihistaminikums, ausgewählt aus der Gruppe bestehend aus Cetirizin, Loratadin und Mischungen derselben,
0,6 - 1,0 Gew.-% Carbopol,
0,2625 - 0,4375 Gew.-% einer mindestens 25 %iger wässrigen Ammoniaklösung,
1,5 - 2,5 Gew.-% Isopropylmyristat,
4,5 - 7,5 Gew.-% Cetiol V,
0,0375 - 0,0625 Gew.-% Natrium-EDTA,
2,25 - 3,75 Gew.-% Macrogol 400,
15,0 - 25,0 Gew.-% 2-Propanol, und
Wasser ad 100 %.

5. Arzneimittelzubereitung gemäß einem der Ansprüche 1 bis 4 zur Therapie einer Erkrankung oder Störung, ausgewählt aus der Gruppe bestehend aus der Urtikaria, allergisch bedingten Dermatosen (allergischen Hautreaktionen), dem atopischen Ekzem, Juckreiz, Rötungen, Sonnenbrand und Insektenstichen.

6. Arzneimittelzubereitung gemäß einem der Ansprüche 1 bis 5, enthaltend zusätzlich einen oder mehrere kosmetische Hilfsstoffe.

7. Arzneimittelzubereitung gemäß Anspruch 6, worin die kosmetischen Hilfsstoffe ausgewählt sind aus der Gruppe, bestehend aus Feuchthaltemitteln, Glättungsmitteln, UV-Filtern, Pigmenten, Farbstoffen, Parfümen, Vitaminen und Bleichmitteln.

8. Arzneimittelzubereitung nach einem der vorstehenden Ansprüche, worin das H1 Antihistaminikum ausgewählt ist unter Cetirizin, Cetirizinhydrochlorid, Loratadin, Loratadinhydrochlorid und Mischungen derselben.
